# EUROPEAN PATENT APPLICATION

(11) **EP 4 332 990 A1**
(43) Date of publication of application: **06.03.2024**
(21) Application number: 22794943.5
(22) Date of filing: 27.04.2022
(51) Int. Cl.: G16H 80/00

(54) **SMART HEALTH MANAGEMENT SYSTEM FOR USE IN TELEMEDICINE SERVICE AND METHOD USED IN SAME**

(30) Priority: 27.04.2021 US 202163180334 P
(71) Applicant: United Crest Healthcare Pte Ltd Company, Shanghai 201103 (CN); Ucrest Berhard, Malaysia, 47800 (MY)
(72) Inventor: EG, Kahyee, Malaysia, 47800 (MY); EG, Jonathanchuanjack, Malaysia, 47800 (MY)
(74) Representative: Haseltine Lake Kempner LLP
(86) International application number: PCT/CN2022/089627
(87) International publication number: WO 2022/228473

(57) **Abstract**

A smart health management system for a telemedicine service at least includes a personalized medical management module, a telemedicine service module, a medical examination and equipment management module and an insurance service module. A medical service demander can provide a medical diagnosis service request through the personalized medical management module. Moreover, the medical service demander and the telemedicine service module perform a telemedicine diagnosis service. Moreover, the medical examination and equipment management module is used to acquire a pathological inspection and imaging examination information. Consequently, the telemedicine service module generates a medical diagnosis information and a drug prescription information in response to the medical examination and equipment management module. Moreover, in response to a medical insurance request and an electronic medical record, the insurance service module generates an insurance claim information automatically.

## Description

### FIELD OF THE INVENTION

The present invention relates to a field of the smart medical management, and more particularly to a smart health management system for a telemedicine service and a smart health management method for a telemedicine service.

### BACKGROUND OF THE INVENTION

Generally, the protocols, programs, processes or methods associated with the conventional or existing medical healthcare systems cannot be cooperatively and effectively operated for the stakeholders (e.g., patients, physicians, hospitals, medical devices/equipment, pharmaceutical companies, pharmacies, insurance companies and the like) in medical healthcare industries. Moreover, since all of these stakeholders disconnect from their own systems and even form independent interest circles, a very closed phenomenon is formed. The respective closure and disconnection results in some drawbacks. For example, the efficiency is low, and the cost is high. Moreover, the divergence of medical data (e.g., electronic medical records) occur when the medical data are transmitted from one stakeholder to another. Moreover, the repeated inspections usually generate serious waste of medical resources.

On the other hand, for various medical devices used in conventional or existing medical healthcare systems, these devices are only used as stand-alone devices, although they are possibly connected to an application (APP) in a smart phone via Bluetooth. Consequently, if one or more batches of medical devices have designing and manufacturing defects, there will be no way to instantly track, identify and contact patients to recall the defective medical devices or notify patients to stop using the defective medical devices. In other words, the medical devices that lack the complete loT functionality will gradually be unsuitable for the future medical field.

Although some systems or architectures claiming to have telemedicine functions have been proposed at present, their functions are still not satisfactory. For example, they usually only have some simple conference or consultation functions, or they are only equipped with some simple physiological detection devices to do some simple physiological data monitoring functions. Moreover, it is impossible to integrate different medical institutions or drug dealers and various pathological inspection units and/or medical imaging service providers across fields. More importantly, it is also unable to integrate the insurance claim functions that must accompany the medical process. In other words, the contents proposed by these so-called telemedicine systems or architectures are not enough to meet the needs of a normal and complete medical service. Moreover, this is the problem that the present invention intends to solve.

Therefore, the present invention provides a smart health management system and a smart health management method for a telemedicine service in order to overcome the drawbacks of the conventional technologies.

### SUMMARY OF THE INVENTION

An object of the present invention provides a smart health management system for a telemedicine service and a smart health management method for a telemedicine service in order to optimize the overall medical process and service content.

In accordance with an aspect of the present invention, a smart health management system for a telemedicine service is provided. The smart health management system includes a medical control module, a telemedicine diagnosis service module, a medical examination and equipment management module, a drug preparation and supply module, an integrated electronic medical record and artificial intelligence analysis data module, an insurance service module and a virtual digital medical institution operation module. The medical control module generates at least one of a medical diagnosis service request, an autonomous health management information, a medical care and information sharing request and a medical insurance request. The telemedicine diagnosis service module is electrically connected with the medical control module. The telemedicine diagnosis service module performs a telemedicine diagnosis service and generates a medical diagnosis information in response to at least one of the medical diagnosis service request and the autonomous health management information. The medical examination and equipment management module is electrically connected with the medical control module and the telemedicine diagnosis service module. The medical examination and equipment management module supports the telemedicine diagnosis service to generate a pathological inspection and imaging examination information and allows the telemedicine diagnosis service module to generate the medical diagnosis information. The drug preparation and supply module is electrically connected with the telemedicine diagnosis service module. The drug preparation and supply module generates a drug prescription information in response to the medical diagnosis information. The drug prescription information is provided to the telemedicine diagnosis service module and the medical control module for use. The integrated electronic medical record and artificial intelligence analysis data module is electrically connected with the medical control module, the telemedicine diagnosis service module, the medical examination and equipment management module and the drug preparation and supply module. The integrated electronic medical record and artificial intelligence analysis data module stores at least one of the autonomous health management information, the medical diagnosis information, the pathological inspection and imaging examination information and the drug prescription information and generates a personalized medical analysis information automatically, or at least one of the telemedicine diagnosis service module and the integrated electronic medical record and artificial intelligence analysis data module provides a medical care and sharing information in response to the medical care and information sharing request from the medical control module. An electronic medical record is automatically generated according to at least one of the autonomous health management information, the medical diagnosis information, the pathological inspection and imaging examination information, the drug prescription information, the personalized medical analysis information and the medical care and sharing information. The insurance service module is electrically connected with the medical control module, the telemedicine diagnosis service module, the medical examination and equipment management module, the drug preparation and supply module and the integrated electronic medical record and artificial intelligence analysis data module. The insurance service module generates an insurance claim information automatically in response to the medical insurance request and the electronic medical record and in response to a cash flow information provided by at least one of the telemedicine diagnosis service module, the medical examination and equipment management module and the drug preparation and supply module. The virtual digital medical institution operation module is electrically connected with the telemedicine diagnosis service module. The virtual digital medical institution operation module supports at least one online medical institution in the telemedicine diagnosis service module to perform the telemedicine diagnosis service.

In an embodiment, the medical control module generates at least one of the medical diagnosis service request, the medical care and information sharing request and the medical insurance request in response to a demand instruction information of at least one user and/or in response to a personalized basic physiological information description provided by the at least one user that at least includes a personal symptom, a personal medical history and a personal family history, or the medical control module generates the autonomous health management information by inputting a physiological characteristic information of the at least one user that is measured or monitored through a physiological feature detection device.

In an embodiment, the medical control module and the at least one user are in wired or wireless communication with each other through a fixed computer or communication device, or the medical control module and the at least one user are in wired or wireless communication with each other through a portable computer or communication device.

In an embodiment, the medical diagnosis service request includes a real-time geographic location that reflects a location of the at least one user.

In an embodiment, the physiological feature detection device at least includes at least one of a sphygmomanometer, an oximeter, a blood glucose meter, a portable electrocardiograph, a body mass index (BMI) meter and a continuous positive airway pressure breathing (CPAP) device.

In an embodiment, the medical control module further includes a contact and trace unit. The contact and trace unit proactively searches for the at least one user that is located in a specific area and proactively communicates with the at least one use.

In an embodiment, the at least one user is in communication between the medical control module and the telemedicine diagnosis service module to perform the telemedicine diagnosis service through a network video and communication software.

In an embodiment, the at least one online medical institution in the telemedicine diagnosis service module includes at least one of an online specialist clinic, an online hospital and an online doctor individual organization from all over the world.

In an embodiment, the medical examination and equipment management module at least includes at least one of a pathological inspection device and an imaging examination device.

In an embodiment, the pathological inspection device at least includes at least one of an electrocardiograph, a bone density testing device, an automatic blood/urine analyzer and a genetic testing device, or the imaging examination device at least includes at least one of an X-ray device, a tomography (CT) scanning device, a nuclear magnetic resonance (MRI) device, a fundus camera and an ultrasound device.

In an embodiment, the medical examination and equipment management module is provided by at least one medical examination and equipment supplier from all over the world.

In an embodiment, the integrated electronic medical record and artificial intelligence analysis data module is installed in one of a wired data storage device and a wireless data storage device, and each of the medical control module, the telemedicine diagnosis service module, the medical examination and equipment management module and the drug preparation and supply module includes a memory card with a wireless data transmission function. If the integrated electronic medical record and artificial intelligence analysis data module is installed in the wireless data storage device, the autonomous health management information, the medical diagnosis information, the pathological inspection and imaging examination information and the drug prescription information are wirelessly transmitted to the wireless data storage device.

In an embodiment, the medical control module includes a main control unit for controlling each of the integrated electronic medical record and artificial intelligence analysis data module, the telemedicine diagnosis service module, the medical examination and equipment management module, the drug preparation and supply module and the insurance service module. Moreover, the medical control module is installed in one of the wired data storage device and the wireless data storage device.

In an embodiment, the integrated electronic medical record and artificial intelligence analysis data module uses an artificial intelligence technology to analyze, integrate or identify at least one of the autonomous health management information, the medical diagnosis information, the pathological inspection and imaging examination information and the drug prescription information so as to generate the personalized medical analysis information automatically.

In an embodiment, the electronic medical record is implemented by using a block chain technology.

In an embodiment, the medical care and sharing information is provided to the at least one user and/or at least one additional user for use. The at least one additional user is selected and designated by the at least one user.

In an embodiment, the at least one user authorizes the at least one additional user to use the electronic medical record in whole or in part.

In an embodiment, the at least one user has a highest management right over the electronic medical record, and the at least one user completely or partially restricts each of the telemedicine diagnosis service module, the medical examination and equipment management module, the drug preparation and supply module and the insurance service module to use the electronic medical record in whole or in part.

In an embodiment, the virtual digital medical institution operation module at least includes at least one of an appointment consultation and examination management function, a customer relationship management (CRM) function and a marketing function so as to support the at least one online medical institution to perform the telemedicine diagnosis service.

In an embodiment, at least one of the medical control module, the telemedicine diagnosis service module, the medical examination and equipment management module, the drug preparation and supply module and the insurance service module has an equipment failure detection function of automatically notifying the at least one user of an equipment failure information.

In an embodiment, at least one of the medical control module, the telemedicine diagnosis service module, the medical examination and equipment management module, the drug preparation and supply module and the insurance service module has an Internet of Things (loT) function.

In an embodiment, the medical diagnosis information, the pathological inspection and imaging examination information and the drug prescription information contain a medical diagnosis cash flow information, a pathological inspection and imaging examination cash flow information and a drug prescription cash flow information, respectively.

In accordance with another aspect of the present invention, a smart health management system for a telemedicine service is provided. The smart health management system includes a personalized medical management module, a telemedicine service module, a medical examination and equipment management module and an insurance service module. The personalized medical management module generates at least one of a medical diagnosis service request, an autonomous health management information, a medical care and information sharing request and a medical insurance request. The telemedicine service module is electrically connected with the personalized medical management module. The telemedicine service module performs a telemedicine diagnosis service and generates a medical diagnosis information in response to at least one of the medical diagnosis service request and the autonomous health management information, or the telemedicine service module provides a medical care and sharing information in response to the medical care and information sharing request. The medical examination and equipment management module is electrically connected with the personalized medical management module and the telemedicine service module. The medical examination and equipment management module supports the telemedicine diagnosis service to generate a pathological inspection and imaging examination information and allows the telemedicine service module to generate the medical diagnosis information. The personalized medical management module stores at least one of the autonomous health management information, the medical diagnosis information, the pathological inspection and imaging examination information and the drug prescription information and generates a personalized medical analysis information automatically. An electronic medical record is automatically generated according to at least one of the autonomous health management information, the medical diagnosis information, the pathological inspection and imaging examination information, the drug prescription information, the personalized medical analysis information and the medical care and sharing information. The insurance service module is electrically connected with the personalized medical management module, the medical examination and equipment management module and the telemedicine service module. The insurance service module generates an insurance claim information automatically in response to the medical insurance request and the electronic medical record and in response to a cash flow information provided by at least one of the medical examination and equipment management module and the telemedicine service module.

In an embodiment, the personalized medical management module at least includes a medical control module and an integrated electronic medical record and artificial intelligence analysis data module. The medical control module generates at least one of the medical diagnosis service request, the autonomous health management information, the medical care and information sharing request and the medical insurance request. The integrated electronic medical record and artificial intelligence analysis data module is electrically connected with the medical control module, the telemedicine service module and the medical examination and equipment management module. The integrated electronic medical record and artificial intelligence analysis data module stores at least one of the autonomous health management information, the medical diagnosis information, the pathological inspection and imaging examination information and the drug prescription information and generates the personalized medical analysis information automatically, or at least one of the telemedicine service module and the integrated electronic medical record and artificial intelligence analysis data module provides a medical care and sharing information in response to the medical care and information sharing request from the medical control module. The electronic medical record is automatically generated according to at least one of the medical diagnosis information, the pathological inspection and imaging examination information, the drug prescription information, the personalized medical analysis information and the medical care and sharing information.

In an embodiment, the medical control module generates at least one of the medical diagnosis service request, the medical care and information sharing request and the medical insurance request in response to a demand instruction information of at least one user and/or in response to a personalized basic physiological information description provided by the at least one user that at least includes a personal symptom, a personal medical history and a personal family history, or the medical control module generates the autonomous health management information by inputting a physiological characteristic information of the at least one user that is measured or monitored through a physiological feature detection device.

In an embodiment, the medical control module and the at least one user are in wired or wireless communication with each other through a fixed computer or communication device, or the medical control module and the at least one user are in wired or wireless communication with each other through a portable computer or communication device.

In an embodiment, the at least one user is in communication between the medical control module and the telemedicine service module to perform the telemedicine diagnosis service through a network video and communication software.

In an embodiment, the integrated electronic medical record and artificial intelligence analysis data module is installed in one of a wired data storage device and a wireless data storage device, and each of the medical control module, the telemedicine service module, the medical examination and equipment management module and the drug preparation and supply module includes a memory card with a wireless data transmission function. If the integrated electronic medical record and artificial intelligence analysis data module is installed in the wireless data storage device, the autonomous health management information, the medical diagnosis information, the pathological inspection and imaging examination information and the drug prescription information are wirelessly transmitted to the wireless data storage device.

In an embodiment, the integrated electronic medical record and artificial intelligence analysis data module uses an artificial intelligence technology to analyze, integrate or identify at least one of the autonomous health management information, the medical diagnosis information, the pathological inspection and imaging examination information and the drug prescription information so as to generate the personalized medical analysis information automatically.

In an embodiment, the telemedicine service module at least includes a telemedicine diagnosis service module and a drug preparation and supply module. The telemedicine diagnosis service module is electrically connected with the personalized medical management module. The telemedicine diagnosis service module performs the telemedicine diagnosis service and generates the medical diagnosis information in response to at least one of the medical diagnosis service request and the autonomous health management information. The drug preparation and supply module is electrically connected with the telemedicine diagnosis service module. The drug preparation and supply module generates the drug prescription information in response to the medical diagnosis information. The drug prescription information is provided to the telemedicine diagnosis service module and the personalized medical management module for use.

In an embodiment, the at least one online medical institution in the telemedicine diagnosis service module includes at least one of an online specialist clinic, an online hospital and an online doctor individual organization from all over the world.

In an embodiment, the medical examination and equipment management module at least includes at least one of a pathological inspection device and an imaging examination device.

In an embodiment, the medical examination and equipment management module is provided by at least one medical examination and equipment supplier from all over the world.

In an embodiment, the medical care and sharing information is provided to the at least one user and/or at least one additional user for use. The at least one additional user is selected and designated by the at least one user.

In an embodiment, the at least one user authorizes the at least one additional user to use the electronic medical record in whole or in part.

In an embodiment, the at least one user has a highest management right over the electronic medical record, and the at least one user completely or partially restricts each of the telemedicine service module, the medical examination and equipment management module and the insurance service module to use the electronic medical record in whole or in part.

In an embodiment, the medical diagnosis information, the pathological inspection and imaging examination information and the drug prescription information contain a medical diagnosis cash flow information, a pathological inspection and imaging examination cash flow information and a drug prescription cash flow information, respectively.

In an embodiment, the smart health management system for the telemedicine service further includes a virtual digital medical institution operation module. The virtual digital medical institution operation module is electrically connected with the telemedicine service module. The virtual digital medical institution operation module supports at least one online medical institution in the telemedicine service module to perform the telemedicine diagnosis service.

In accordance with another aspect of the present invention, a smart health management method for a telemedicine service is provided. The smart health management method at least includes the following steps. Firstly, at least one of a medical diagnosis service request, an autonomous health management information, a medical care and information sharing request and a medical insurance request is provided. Then, a telemedicine diagnosis service is performed and a medical diagnosis information and a drug prescription information are generated in response to at least one of the medical diagnosis service request and the autonomous health management information, or providing a medical care and sharing information in response to the medical care and information sharing request. Then, the telemedicine diagnosis service is supported to generate a pathological inspection and imaging examination information. Then, a medical diagnosis information is generated in response to the pathological inspection and imaging examination information. Then, a personalized medical analysis information is generated in response to at least one of the autonomous health management information, the medical diagnosis information, the pathological inspection and imaging examination information and the drug prescription information, and an electronic medical record is generated automatically according to at least one of the autonomous health management information, the medical diagnosis information, the pathological inspection and imaging examination information, the drug prescription information, the personalized medical analysis information and the medical care and sharing information. Then, an insurance claim information is generated automatically in response to the medical insurance request and the electronic medical record.

In an embodiment, at least one of the medical diagnosis service request, the medical care and information sharing request and the medical insurance request is generated in response to a demand instruction information of at least one user and/or in response to a personalized basic physiological information description provided by the at least one user that at least includes a personal symptom, a personal medical history and a personal family history, or the autonomous health management information is generated by measuring or monitoring a physiological characteristic information of the at least one user.

In an embodiment, the at least one user performs the telemedicine diagnosis service through a network video and communication software.

In an embodiment, the telemedicine diagnosis service is provided by at least one of an online specialist clinic, an online hospital and an online doctor individual organization from all over the world.

In an embodiment, the pathological inspection and imaging examination information is provided by at least one medical examination and equipment supplier from all over the world.

In an embodiment, the medical diagnosis service request includes a real-time geographic location that reflects a location of the at least one user.

In an embodiment, the smart health management method further includes a step of proactively searching for the at least one user that is located in a specific area and proactively communicating with the at least one use.

In an embodiment, the smart health management system further includes a step of using an artificial intelligence technology to analyze, integrate or identify at least one of the autonomous health management information, the medical diagnosis information, the pathological inspection and imaging examination information and the drug prescription information so as to generate the personalized medical analysis information automatically.

In an embodiment, the electronic medical record is implemented by using a block chain technology.

In an embodiment, the medical care and sharing information is provided to the at least one user and/or at least one additional user for use. The at least one additional user is selected and designated by the at least one user.

In an embodiment, the at least one user authorizes the at least one additional user to use the electronic medical record in whole or in part.

In an embodiment, the at least one user has a highest management right over the electronic medical record, and the at least one user completely or partially restricts a use access right and/or a modification right of any module or the at least one additional user on the electronic medical record.

The above embodiments of the present invention at least have the following beneficial effects. For example, the smart health management system and the smart health management method can provide the user with real-time telehealth service or guidance, optimize the overall medical process and service content, avoid a large number of repeated physical examinations or imaging examinations, or reduce the time period of repeatedly confirming the medical history. Consequently, it can be ensured that the core concept for the medical service demanders can be concretely implemented.

### BRIEF DESCRIPTION OF THE DRAWINGS

In order to illustrate the technical solutions of the embodiments of the present invention more clearly, the accompanying drawings that are used in the description of the embodiments will be briefly introduced as follows. Obviously, the accompanying drawings in the following description are only some embodiments of the present invention. For those of ordinary skill in the art, other drawings can also be obtained from these drawings without any creative effort.
FIG. 1 is a schematic functional block diagram illustrating the concept of a smart health management system for a telemedicine service according to an embodiment of the present invention.
FIG. 2 is a schematic functional block diagram illustrating an implementation example of the smart health management system for a telemedicine service according to the embodiment of the present invention.
FIG. 3 is a schematic functional block diagram illustrating some functions of the smart health management system as shown in FIG. 2 in response to the medical care and information sharing request.
FIG. 4 is a flowchart illustrating a smart health management method for a telemedicine service according to an embodiment of the present invention.

### DESCRIPTION OF THE MAIN ELEMENT SYMBOLS:

1: smart health management system; 11: personalized medical management module; 111: medical control module; 111: main control unit; 1112: contact and trace unit; 112: integrated electronic medical record and artificial intelligence analysis data module; 12: telemedicine service module; 120: virtual digital medical institution operation module; 121: telemedicine diagnosis service module; 122: drug preparation and supply module; 13: medical examination and equipment management module; 131: pathological inspection device; 132: imaging examination device; 14: insurance service module; 15: physiological feature detection device; B: personalized basic physiological information description; E: electronic medical record; I: demand instruction information; P1: at least one user; P2: at least one additional user; R1: medical diagnosis service request; R2: medical care and information sharing request; R3: medical insurance request; S1: autonomous health management information; S11: physiological characteristic information; S2: medical diagnosis information; S20: medical diagnosis cash flow information; S3: pathological inspection and imaging examination information; S30: pathological inspection and imaging examination cash flow information; S4: drug prescription information; S40: drug prescription cash flow information; S5: personalized medical analysis information; S6: medical care and sharing information; S7: insurance claim information; S8: equipment failure information.

### DETAILED DESCRIPTION OF THE PREFERRED EMBODIMENT

The technical solutions in the embodiments of the present invention will be clearly and completely described below with reference to the accompanying drawings in the embodiments of the present invention. Obviously, the described embodiments are only a part of the embodiments of the present invention, rather than all of the embodiments. All other embodiments obtained by those of ordinary skill in the art without creative work will fall within the protected scopes of the present invention.

It is noted that the terms "upper", "lower", "front" and "rear" in the description and claims of this application are only used to describe and understand this application, but not to limit this application. Moreover, in the specification, unless explicitly described to the contrary, the word "comprising" will be understood to mean the inclusion of stated elements, but not the exclusion of any other elements.

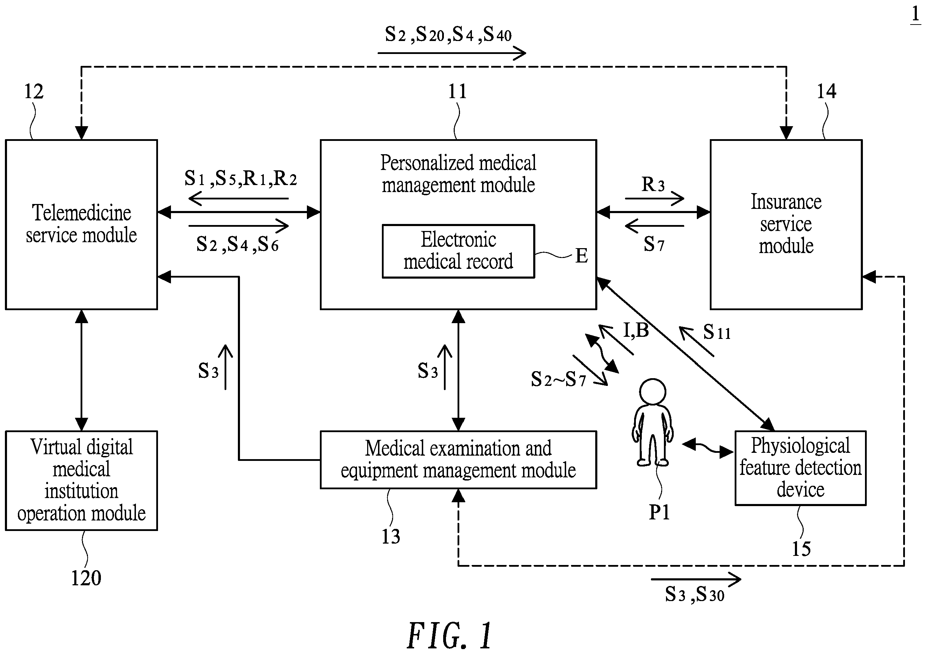

FIG. 1 is a schematic functional block diagram illustrating the concept of a smart health management system for a telemedicine service according to an embodiment of the present invention. The smart health management system 1 (which can also be regarded as a medical service provider) at least comprises a personalized medical management module 11, a telemedicine service module 12, a medical examination and equipment management module 13 and an insurance service module 14.

Preferably but not exclusively, the personalized medical management module 11 is used for generating at least one of a medical diagnosis service request R1, an autonomous health management information S1, a medical care and information sharing request R2 and a medical insurance request R3.

Moreover, in response to a demand instruction information I of at least one user P1 (which can also be regarded as a medical service demander) and/or in response to a personalized basic physiological information description B provided by the at least one user P1 (which at least includes a personal symptom, a personal medical history and a personal family history), the personalized medical management module 11 generates at least one of the medical diagnosis service request R1, the medical care and information sharing request R2 and the medical insurance request R3. Alternatively, by inputting a physiological characteristic information of the at least one user P1 that is measured or monitored through a physiological feature detection device 15, the personalized medical management module 11 generates the autonomous health management information S1.

In an embodiment, the physiological feature detection device 15 at least comprises at least one of a sphygmomanometer, an oximeter, a blood glucose meter, a portable electrocardiograph, a body mass index (BMI) meter and a continuous positive airway pressure breathing (CPAP) device. It is noted that the example of the physiological feature detection device 15 is not restricted.

Of course, the personalized medical management module 11 and the at least one user P1 can be in wired or wireless communication with each other through a fixed computer or communication device (not shown), or the personalized medical management module 11 and the at least one user P1 can be in wired or wireless communication with each other through a portable computer or communication device (not shown). For example, the personalized medical management module 11 is installed in a cloud service platform. Under this circumstance, the at least one user P1 can be in network communication with the personalized medical management module 11 through a desktop computer, a notebook computer or a mobile phone in a wired or wireless transmission manner.

The telemedicine service module 12 is electrically connected with the personalized medical management module 11. In response to at least one of the medical diagnosis service request R1 and the autonomous health management information S1, the telemedicine service module 12 performs a telemedicine diagnosis service and generates a medical diagnosis information S2 and a drug prescription information S4. Moreover, in response to the medical care and information sharing request R2, the telemedicine service module 12 provides a medical care and sharing information S6.

Of course, the drug prescription information S4 is a medical information that is generated according to the medical diagnosis information S2.

By using a network video and communication software, the at least one user P1 and the telemedicine service module 12 can be in communication with each other to perform the telemedicine diagnosis service. This telemedicine diagnosis service is provided by at least one online medical institution in the telemedicine service module. The at least one online medical institution includes at least one of online specialist clinics, online hospitals and online doctor individual organizations from all over the world.

Of course, the example of the at least one online medical institution is not restricted. For example, in some other embodiments, the at least one online medical institution further includes at least one online pharmaceutical factory or at least one online pharmacy.

The at least one user P1 can freely and conveniently select or designate a suitable person from online medical resources around the world to perform the telemedicine diagnosis service. Consequently, the concept of a global digital hospital can be realized.

Moreover, in order to meet and provide administrative and operational support for medical organizations or medical individuals from all over the world, the smart health management system 1 further include a virtual digital medical institution operation module 120. The virtual digital medical institution operation module 120 is electrically connected with the telemedicine service module 12. The virtual digital medical institution operation module 120 is used for supporting the at least one online medical institution in the telemedicine service module to perform the telemedicine diagnosis service.

The virtual digital medical institution operation module 120 at least comprises at least one of an appointment consultation and examination management function, a customer relationship management (CRM) function and a marketing function in order to support the at least one online medical institution to perform the telemedicine diagnosis service.

The medical care and sharing information S6 may be referred to a telehealth service that the telemedicine service module 12 provides to the at least one user P1 after receiving the medical care and information sharing request R2. For example, if the at least one user P1 has a long-term chronic disease history, the telemedicine service module 12 can proactively notify the at least one user P1 to take medicine or see the medicine or provide any other appropriate medical care reminder regularly or irregularly through the medical care and sharing information S6. Moreover, the telemedicine service module 12 can also grasp the physiological status of the at least one user P1 in real time through the returned autonomous health management information S1 and provide the at least one user P1 with real-time telehealth service or guidance through the medical care and sharing information S6.

Another key implementation module of the present invention is the medical examination and equipment management module 13. The medical examination and equipment management module 13 is electrically connected with the personalized medical management module 11 and the telemedicine service module 12. The medical examination and equipment management module 13 is used for supporting the telemedicine diagnosis service to generate a pathological inspection and imaging examination information S3 and allowing the telemedicine service module 12 to generate the medical diagnosis information S2.

In an embodiment, the pathological inspection and imaging examination information S3 is provided by at least one medical examination and equipment supplier from all over the world.

For example, during the process of the telemedicine diagnosis service, the at least one user P1 can passively accept the designation of the medical examination and equipment management module 13, or the at least one user P1 can proactively select various types of medical institutions near the home from the list and/or select pathological examination centers and imaging examination centers that are independently operated. Consequently, the required pathological examinations and imaging examinations can be completed. Then, the medical examination and equipment management module 13 will send the generated pathological inspection and imaging examination information S3 back to the telemedicine service module 12 and the at least one user P1. According to the pathological inspection and imaging examination information S3, the telemedicine service module 12 generates the medical diagnosis information S2.

Of course, if some pathological examinations and imaging examinations can be carried out online, they can also be carried out through the smart health management system 1 of this embodiment.

In accordance with another feature of the present invention, the personalized medical management module 11 is used for storing at least one of the autonomous health management information S1, the medical diagnosis information S2, the pathological inspection and imaging examination information S3 and the drug prescription information S4. Moreover, the personalized medical management module 11 generates a personalized medical analysis information S5 according to at least one of the autonomous health management information S1, the medical diagnosis information S2, the pathological inspection and imaging examination information S3 and the drug prescription information S4 that are stored. Moreover, at least one of the autonomous health management information S1, the medical diagnosis information S2, the pathological inspection and imaging examination information S3, the drug prescription information S4, the personalized medical analysis information S5 and the medical care and sharing information S6 can be used to generate an electronic medical record E automatically.

In an embodiment, an artificial intelligence technology can be used to analyze, integrate or identify at least one of the autonomous health management information S1, the medical diagnosis information S2, the pathological inspection and imaging examination information S3 and the drug prescription information S4 in order to generate the personalized medical analysis information S5 automatically.

That is, for a large number of electronic medical records E, they constitute a large data database by themselves. Consequently, a machine learning process and a data mining process can be carried out through the aforementioned artificial intelligence technology to optimize the overall medical process and service content, which will be provided to the medical service providers and the medical service demanders of the present invention for use.

Furthermore, the electronic medical record E can also be implemented by using a block chain technology. Consequently, it can be fully ensured that the medical data will not be arbitrarily tampered, or the non-repudiation of the data content can be strengthened.

In this way, the electronic medical record E can be collaboratively authenticated and used by the telemedicine service module 12, the medical examination and equipment management module 13 and the insurance service module 14. Consequently, when the at least one user P1 uses medical services provided by different medical institutions, the problems associated with the exchange of the case data can be avoided. Moreover, a large number of repeated physical examinations or imaging examinations can be avoided, and time period of repeatedly confirming the medical history can be reduced.

Moreover, another embodiment of the present invention provides a system design that closely integrates medical resources and insurance resources. As shown in FIG. 1, the insurance service module 14 is electrically connected with the personalized medical management module 11, the medical examination and equipment management module 13 and the telemedicine service module 12. In response to the medical insurance request R3 and the electronic medical record E and in response to the cash flow information provided by at least one of the medical examination and equipment management module 13 and the telemedicine service module 12, the insurance service module 14 generates an insurance claim information S7 automatically.

The abovementioned cash flow information can be included in the medical diagnosis information S2, the pathological inspection and imaging examination information S3 and the drug prescription information S4. The cash flow information can be a medical diagnosis cash flow information S20, a pathological inspection and imaging examination cash flow information S30 and a drug prescription cash flow information S40, respectively. In another embodiment, each of the medical diagnosis cash flow information S20, the pathological inspection and imaging examination cash flow information S30 and the drug prescription cash flow information S40 is an independent cash flow information. That is, the medical diagnosis cash flow information S20, the pathological inspection and imaging examination cash flow information S30 and the drug prescription cash flow information S40 are not included in the medical diagnosis information S2, the pathological inspection and imaging examination information S3 and the drug prescription information S4, respectively.

In an embodiment, at least one of the personalized medical management module 11, the telemedicine service module 12, the medical examination and equipment management module 13 and the insurance service module 14 as shown in FIG. 1 has an Internet of Things (loT) function.

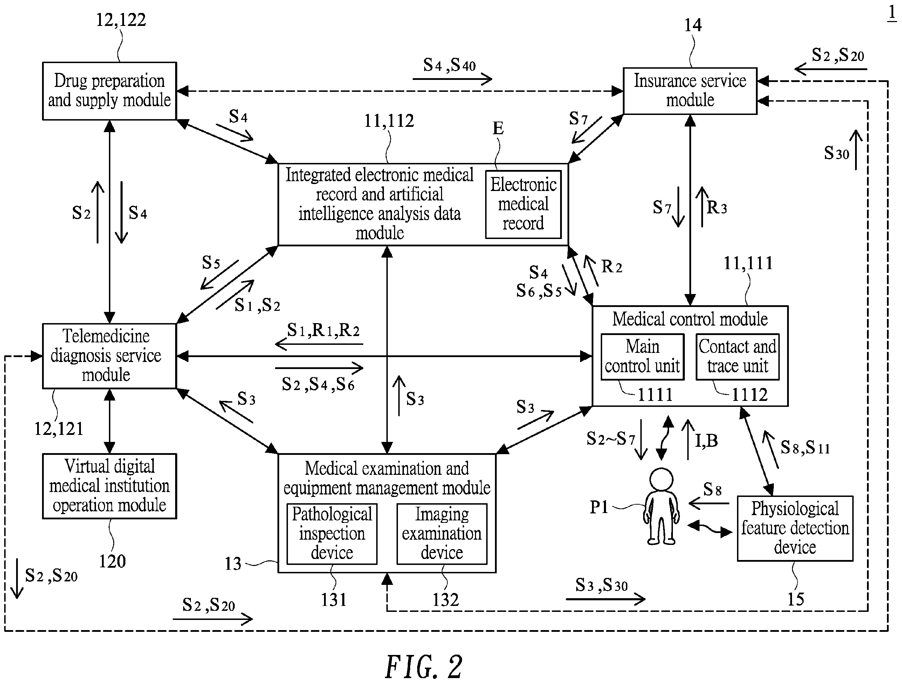

FIG. 2 is a schematic functional block diagram illustrating an implementation example of the smart health management system for a telemedicine service according to the embodiment of the present invention. It is noted that the implementation examples are not restricted.

Please refer to FIG. 1 and FIG. 2. In comparison with the architecture of FIG. 1, the personalized medical management module 11 as shown in FIG. 2 at least comprises a medical control module 111 and an integrated electronic medical record and artificial intelligence analysis data module 112, and the telemedicine service module 12 as shown in FIG. 2 at least comprises a telemedicine diagnosis service module 121 and a drug preparation and supply module 122.

The medical control module 111 is used for generating at least one of the medical diagnosis service request R1, the autonomous health management information S1, the medical care and information sharing request R2 and the medical insurance request R3. The telemedicine diagnosis service module 121 is electrically connected with the medical control module 11. In response to at least one of the medical diagnosis service request R1 and the autonomous health management information S1, the telemedicine diagnosis service module 121 provides a telemedicine diagnosis service and generates the medical diagnosis information S2.

The virtual digital medical institution operation module 120 is electrically connected with the telemedicine diagnosis service module 121. The virtual digital medical institution operation module 120 is used for supporting the at least one online medical institution in the telemedicine diagnosis service module 121 to perform the telemedicine diagnosis service.

Moreover, the drug preparation and supply module 122 is electrically connected with the telemedicine diagnosis service module 121. In response to the medical diagnosis information S2, the drug preparation and supply module 122 generates the drug prescription information S4. Moreover, the drug prescription information S4 is provided to the telemedicine diagnosis service module 121 and the medical control module 111 for use.

The integrated electronic medical record and artificial intelligence analysis data module 112 is electrically connected with the medical control module 111, the telemedicine diagnosis service module 121, the medical examination and equipment management module 13 and the drug preparation and supply module 122. The integrated electronic medical record and artificial intelligence analysis data module 112 is used for storing at least one of the autonomous health management information S1, the medical diagnosis information S2, the pathological inspection and imaging examination information, and the drug prescription information S3. Moreover, integrated electronic medical record and artificial intelligence analysis data module 112 automatically generates the personalized medical analysis information S5 according to at least one of the autonomous health management information S1, the medical diagnosis information S2, the pathological inspection and imaging examination information, and the drug prescription information S3. Alternatively, in response to the medical care and information sharing request R2 from the medical control module 11, at least one of the telemedicine diagnosis service module 121 and the integrated electronic medical record and artificial intelligence analysis data module 112 provides the medical care and sharing information S6.

In an embodiment, the integrated electronic medical record and artificial intelligence analysis data module 112 is installed in one of a wired data storage device (not shown) and a wireless data storage device (not shown). Each of the medical control module 111, the telemedicine diagnosis service module 121, the medical examination and equipment management module 13 and the drug preparation and supply module 122 is equipped with a memory card with a wireless data transmission function (not shown). Consequently, in case that the integrated electronic medical record and artificial intelligence analysis data module 112 is installed in the wireless data storage device, the autonomous health management information S1, the medical diagnosis information S2, the pathological inspection and imaging examination information S3 and the drug prescription information S4 can be wirelessly transmitted to the wireless data storage device.

As shown in FIG. 2, the medical control module 111 comprises a main control unit 1111 for controlling each of the integrated electronic medical record and artificial intelligence analysis data module 112, the telemedicine diagnosis service module 121, the medical examination and equipment management module 13, the drug preparation and supply module 122 and the insurance service module 14. Moreover, the medical control module 111 is installed in one of the wired data storage device and the wireless data storage device.

Consequently, through the medical control module 111 in one of the wired data storage device and the wireless data storage device, the at least one user P1 can be in network communication with the medical control module 111 through a desktop computer, a notebook computer or a mobile phone in a wired or wireless transmission manner.

Please refer to FIG. 2 again. In a more preferred embodiment, the medical control module 111 further comprises a contact and trace unit 1112. The contact and trace unit 1112 is used to proactively search for the at least one user P1 that is located in a specific area and proactively communicate with the at least one user P1.

In this way, the medical diagnosis service request R1 may include a real-time geographic location that can reflect the location of the at least one user P1, and the telemedicine diagnosis service module 121 may proactively provide an instant and proactive medical service to the at least one user P1 who has received the medical service of the present invention. For example, the telemedicine diagnosis service module 121 can proactively send or push a health information and a medication reminder to the at least one user P1. Moreover, by acquiring a physiological characteristic information S11 of the at least one user P1 in real time and combining with the real-time geographic location where the at least one user P1 is located, the telemedicine diagnosis service module 121 can proactively provide emergency medical rescue services when necessary.

The physiological feature detection device 15 as shown in FIG. 2 is used for measuring or monitoring the physiological characteristic information S11 that is generated and outputted by the at least one user P1. According to the physiological characteristic information S11, the medical control module 111 generates the autonomous health management information S1. Moreover, the physiological feature detection device 15 can also be added with an equipment failure detection function. Consequently, in case that the physiological feature detection device 15 malfunctions, the physiological feature detection device 15 can automatically notify at least one of the at least one user P1 and the medical control module 111 of an equipment failure information S8. Consequently, the physiological feature detection device 15 can be further arranged to be sent for repair and maintenance, and the correct physiological characteristic information S11 are re-inputted.

Similarly, at least one of the medical control module 111, the telemedicine diagnosis service module 121, the medical examination and equipment management module 13, the drug preparation and supply module 122 and the insurance service module 14 is also provided with the abovementioned equipment failure detection function. Consequently, the stability of medical equipment and the accuracy of medical data are enhanced.

At least one of the medical control module 111, the telemedicine diagnosis service module 121, the medical examination and equipment management module 13, the drug preparation and supply module 122 and the insurance service module 14 has an Internet of Things (loT) function for performing a large data analysis and artificial intelligence management.

Moreover, the insurance service module as shown in FIG. 2 is electrically connected with the medical control module 111, the telemedicine diagnosis service module 121, the medical examination and equipment management module 13, the drug preparation and supply module 122 and the integrated electronic medical record and artificial intelligence analysis data module 112. In response to the medical insurance request R2 and the electronic medical record E and in response to the cash flow information provided by at least one of the telemedicine diagnosis service module 121, the medical examination and equipment management module 13 and the drug preparation and supply module 122, the insurance service module 14 generates the insurance claim information S7 automatically.

The abovementioned cash flow information can be included in the medical diagnosis information S2, the pathological inspection and imaging examination information S3 and the drug prescription information S4. The cash flow information can be a medical diagnosis cash flow information S20, a pathological inspection and imaging examination cash flow information S30 and a drug prescription cash flow information S40, respectively. In another embodiment, each of the medical diagnosis cash flow information S20, the pathological inspection and imaging examination cash flow information S30 and the drug prescription cash flow information S40 is an independent cash flow information. That is, the medical diagnosis cash flow information S20, the pathological inspection and imaging examination cash flow information S30 and the drug prescription cash flow information S40 are not included in the medical diagnosis information S2, the pathological inspection and imaging examination information S3 and the drug prescription information S4, respectively.

Moreover, as shown in FIG. 2, the medical examination and equipment management module 13 at least further comprises at least one of a pathological inspection device 131 and an imaging examination device 132.

In an embodiment, the pathological inspection device 131 at least comprises at least one of an electrocardiograph, a bone density testing device, an automatic blood/urine analyzer and a genetic testing device, or the imaging examination device 132 at least comprises at least one of an X-ray device, a tomography (CT) scanning device, a nuclear magnetic resonance (MRI) device, a fundus camera and an ultrasound device. It is noted that the examples of the pathological inspection device 131 and the imaging examination device 132 are not restricted.

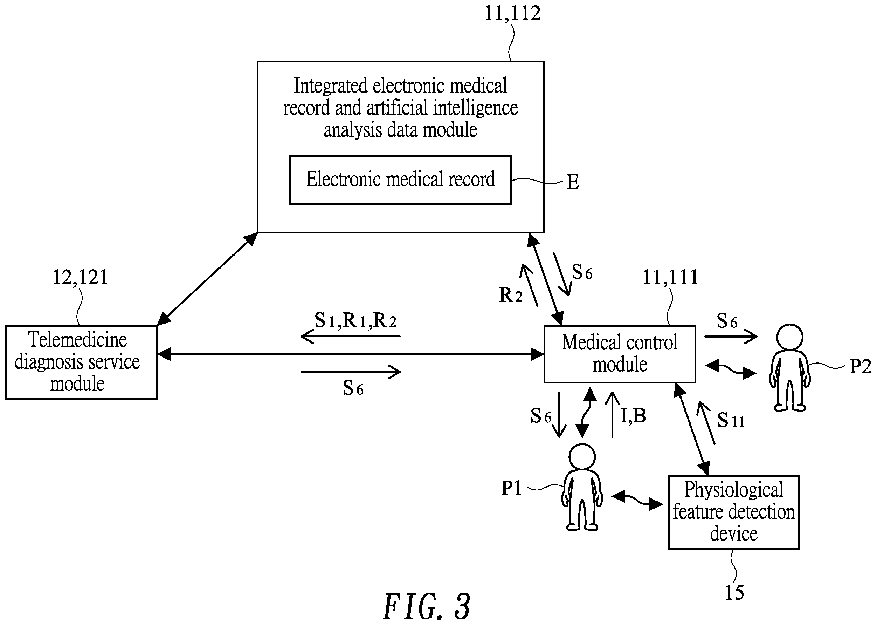

In accordance with the core concept of the present invention, the medical requesters are the service centers. FIG. 3 is a schematic functional block diagram illustrating some functions of the smart health management system as shown in FIG. 2 in response to the medical care and information sharing request R2.

For clearly describing the concepts of the present invention, only portions of the function modules of FIG. 2 (e.g., the medical control module 111, the integrated electronic medical record and artificial intelligence analysis data module 112 and the telemedicine diagnosis service module 121) are shown in FIG. 3. Moreover, the medical care and sharing information S6 is provided to the at least one user P1 and/or at least one additional user P2 for use. The at least one additional user P2 is selected and designated by the at least one user P1. For example, the at least one additional user P2 is a relative, a friend or a medical consultant that is trusted by the at least one user P1, or the at least one additional user P2 is a medical staff.

The at least one user P1 can authorize the at least one additional user P2 to use the electronic medical record in whole or in part. Moreover, the at least one user P1 has the highest management right over the electronic medical record. The at least one user P1 can completely or partially restrict a use access right and/or a modification right of the electronic medical record E.

In case that the at least one additional user P2 is a relative, a friend or a medical consultant trusted by the at least one user P1 or the at least one additional user P2 is a medical staff, a caring and nursing concept is concretely implemented. In case that the at least one user P1 authorizes the at least one additional user P2 to use the electronic medical record E in whole or in part, the concept of sharing individual medical experience with more people can be realized. These special functions are helpful to further optimize the operability, affinity and foresight of the concept of the present invention.

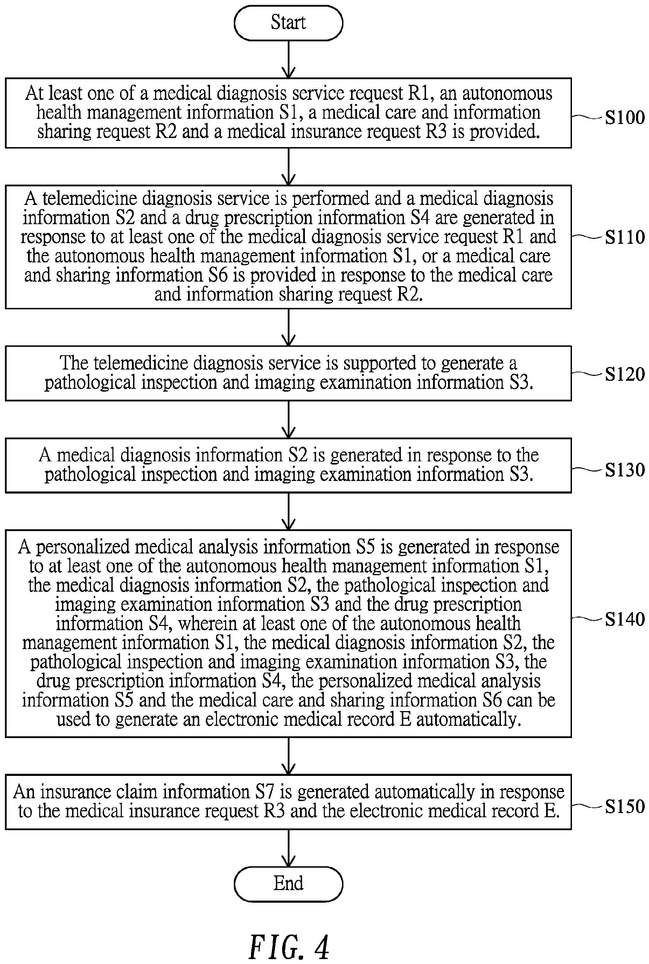

FIG. 4 is a flowchart illustrating a smart health management method for a telemedicine service according to an embodiment of the present invention. For clearly illustrating the implementation of the smart health management method, relevant descriptions will be made in conjunction with the aforementioned embodiments in FIGS. 1 to 3. As shown in FIG. 4, the smart health management method at least comprises the following steps.

In a step S100, at least one of a medical diagnosis service request R1, an autonomous health management information S1, a medical care and information sharing request R2 and a medical insurance request R3 is provided.

In a step S110, a telemedicine diagnosis service is performed and a medical diagnosis information S2 and a drug prescription information S4 are generated in response to at least one of the medical diagnosis service request R1 and the autonomous health management information S1, or a medical care and sharing information S6 is provided in response to the medical care and information sharing request R2.

In a step S120, the telemedicine diagnosis service is supported to generate a pathological inspection and imaging examination information S3.

In a step S130, a medical diagnosis information S2 is generated in response to the pathological inspection and imaging examination information S3.

In a step S140, a personalized medical analysis information S5 is generated in response to at least one of the autonomous health management information S1, the medical diagnosis information S2, the pathological inspection and imaging examination information S3 and the drug prescription information S4. Moreover, at least one of the autonomous health management information S1, the medical diagnosis information S2, the pathological inspection and imaging examination information S3, the drug prescription information S4, the personalized medical analysis information S5 and the medical care and sharing information S6 can be used to generate an electronic medical record E automatically.

In a step S150, an insurance claim information S7 is generated automatically in response to the medical insurance request R3 and the electronic medical record E.

Moreover, in response to a demand instruction information I of at least one user P1 and/or in response to a personalized basic physiological information description B provided by the at least one user P1 (which at least includes a personal symptom, a personal medical history and a personal family history), at least one of the medical diagnosis service request R1, the medical care and information sharing request R2 and the medical insurance request R3 is generated. Alternatively, in response to a physiological characteristic information S11 that is generated by measuring or monitoring the at least one user P1, the autonomous health management information S1 is generated.

By using a network video and communication software, the at least one user P1 can perform the telemedicine diagnosis service. This telemedicine diagnosis service is provided by at least one of an online specialist clinic, an online hospital and an online doctor individual organizations from all over the world. Moreover, the pathological inspection and imaging examination information S3 is provided by at least one medical examination and equipment supplier from all over the world.

The medical diagnosis service request may include a real-time geographic location that can reflect the location of the at least one user P1, or proactively search for the at least one user P1 that is located in a specific area and proactively communicate with the at least one user P1.

Consequently, according to the technologies of the present invention, an instant and proactive medical service can be proactively issued and provided to the at least one user P1 who has received the medical service of the present invention. For example, a health information and a medication reminder can be proactively sent or pushed to the at least one user P1. Moreover, by acquiring the physiological characteristic information S11 of the at least one user P1 in real time and combining with the real-time geographic location where the at least one user P1 is located, emergency medical rescue services can be provided when necessary.

The smart health management method further uses an artificial intelligence technology to analyze, integrate or identify at least one of the autonomous health management information S1, the medical diagnosis information S2, the pathological inspection and imaging examination information S3 and the drug prescription information S4 in order to generate the personalized medical analysis information S5 automatically.

That is, for a large number of electronic medical records E, they constitute a large data database by themselves. Consequently, a machine learning process and a data mining process can be carried out through the aforementioned artificial intelligence technology to optimize the overall medical process and service content, which will be provided to the medical service providers and the medical service demanders of the present invention for use.

Furthermore, the electronic medical record E can be implemented by using a block chain technology. Consequently, it can be fully ensured that the medical data will not be arbitrarily tampered, or the non-repudiation of the data content can be strengthened.

Moreover, the medical care and sharing information is provided to the at least one user P1 and/or at least one additional user P2 for use. The at least one additional user P2 is selected and designated by the at least one user P1. For example, the at least one additional user P2 is a relative, a friend or a medical consultant that is trusted by the at least one user P1, or the at least one additional user P2 is a medical staff.

The at least one user P1 can authorize the at least one additional user P2 to use the electronic medical record E in whole or in part. Moreover, the at least one user P1 has the highest management right over the electronic medical record. The at least one user P1 can completely or partially restrict a use access right and/or a modification right of the electronic medical record E.

The main object of the design is based on the core concept of the present invention. That is, in the smart health management system and the smart health management method of the present invention, the medical requesters are the service centers. Consequently, any medical information (e.g., the electronic medical record E) that is directly and exclusively related to the medical service demander and produced in the smart health management system and the smart health management method of the present invention can be specially set. According to the settings, the medical service demander can determine whether a use access right and/or a modification right of the own medical information (e.g., the electronic medical record E) is completely or partially restricted. Consequently, the personal privacy of those in need of medical services can be protected from being easily leaked or abused.

From the above descriptions, through the above embodiments, it can be ensured that the core concept for the medical service demanders can be concretely implemented. In other words, the technologies of the present invention are industrially valuable.

While the invention has been described in terms of what is presently considered to be the most practical and preferred embodiments, it is to be understood that the invention needs not be limited to the disclosed embodiments. On the contrary, it is intended to cover various modifications and similar arrangements included within the spirit and scope of the appended claims which are to be accorded with the broadest interpretation so as to encompass all modifications and similar structures.

## Claims

1. A smart health management system (1) for a telemedicine service, **characterized in that** the smart health management system (1) comprises:
a medical control module (111) generating at least one of a medical diagnosis service request (R1), an autonomous health management information (S1), a medical care and information sharing request (R2) and a medical insurance request (R3);
a telemedicine diagnosis service module (121) electrically connected with the medical control module (111), wherein the telemedicine diagnosis service module (121) performs a telemedicine diagnosis service and generates a medical diagnosis information (S2) in response to at least one of the medical diagnosis service request (R1) and the autonomous health management information (S1);
a medical examination and equipment management module (13) electrically connected with the medical control module (111) and the telemedicine diagnosis service module (121), wherein the medical examination and equipment management module (13) supports the telemedicine diagnosis service to generate a pathological inspection and imaging examination information (S3) and allows the telemedicine diagnosis service module (121) to generate the medical diagnosis information (S2);
a drug preparation and supply module (122) electrically connected with the telemedicine diagnosis service module (121), wherein the drug preparation and supply module (122) generates a drug prescription information (S4) in response to the medical diagnosis information (S2), and the drug prescription information (S4) is provided to the telemedicine diagnosis service module (121) and the medical control module (111) for use;
an integrated electronic medical record and artificial intelligence analysis data module (112) electrically connected with the medical control module (111), the telemedicine diagnosis service module (121), the medical examination and equipment management module (13) and the drug preparation and supply module (122), wherein the integrated electronic medical record and artificial intelligence analysis data module (112) stores at least one of the autonomous health management information (S1), the medical diagnosis information (S2), the pathological inspection and imaging examination information (S3) and the drug prescription information (S4) and generates a personalized medical analysis information (S5) automatically, or at least one of the telemedicine diagnosis service module (121) and the integrated electronic medical record and artificial intelligence analysis data module (112) provides a medical care and sharing information (S6) in response to the medical care and information sharing request (R2) from the medical control module (111), wherein an electronic medical record (E) is automatically generated according to at least one of the autonomous health management information (S1), the medical diagnosis information (S2), the pathological inspection and imaging examination information (S3), the drug prescription information (S4), the personalized medical analysis information (S5) and the medical care and sharing information (S6);
an insurance service module (S4) electrically connected with the medical control module (111), the telemedicine diagnosis service module (121), the medical examination and equipment management module (13), the drug preparation and supply module (122) and the integrated electronic medical record and artificial intelligence analysis data module (112), wherein the insurance service module (S4) generates an insurance claim information (S7) automatically in response to the medical insurance request (R3) and the electronic medical record (E) and in response to a cash flow information provided by at least one of the telemedicine diagnosis service module (121), the medical examination and equipment management module (13) and the drug preparation and supply module (122); and
a virtual digital medical institution operation module (120) electrically connected with the telemedicine diagnosis service module (121), wherein the virtual digital medical institution operation module (120) supports at least one online medical institution in the telemedicine diagnosis service module (121) to perform the telemedicine diagnosis service.

2. The smart health management system (1) for the telemedicine service according to claim 1, wherein the medical control module (111) generates at least one of the medical diagnosis service request (R1), the medical care and information sharing request (R2) and the medical insurance request (R3) in response to a demand instruction information (I) of at least one user (P1) and/or in response to a personalized basic physiological information description provided by the at least one user (P1) that at least includes a personal symptom, a personal medical history and a personal family history, or the medical control module (111) generates the autonomous health management information (S1) by inputting a physiological characteristic information (S11) of the at least one user (P1) that is measured or monitored through a physiological feature detection device (15).

3. The smart health management system (1) for the telemedicine service according to claim 2, wherein the medical control module (111) and the at least one user (P1) are in wired or wireless communication with each other through a fixed computer or communication device, or the medical control module (111) and the at least one user (P1) are in wired or wireless communication with each other through a portable computer or communication device.

4. The smart health management system (1) for the telemedicine service according to claim 3, wherein the medical diagnosis service request (R1) includes a real-time geographic location that reflects a location of the at least one user (P1).

5. The smart health management system (1) for the telemedicine service according to claim 2, wherein the physiological feature detection device (15) at least comprises at least one of a sphygmomanometer, an oximeter, a blood glucose meter, a portable electrocardiograph, a body mass index (BMI) meter and a continuous positive airway pressure breathing (CPAP) device.

6. The smart health management system (1) for the telemedicine service according to claim 2, wherein the medical control module (111) further comprises a contact and trace unit (1112), wherein the contact and trace unit (1112) proactively searches for the at least one user (P1) that is located in a specific area and proactively communicates with the at least one use.

7. The smart health management system (1) for the telemedicine service according to claim 2, wherein the at least one user (P1) is in communication between the medical control module (111) and the telemedicine diagnosis service module (121) to perform the telemedicine diagnosis service through a network video and communication software.

8. The smart health management system (1) for the telemedicine service according to claim 1, wherein the at least one online medical institution in the telemedicine diagnosis service module (121) includes at least one of an online specialist clinic, an online hospital and an online doctor individual organization from all over the world.

9. The smart health management system (1) for the telemedicine service according to claim 1, wherein the medical examination and equipment management module (13) at least comprises at least one of a pathological inspection device (131) and an imaging examination device (132).

10. The smart health management system (1) for the telemedicine service according to claim 9, wherein the pathological inspection device (131) at least comprises at least one of an electrocardiograph, a bone density testing device, an automatic blood/urine analyzer and a genetic testing device, or the imaging examination device (132) at least comprises at least one of an X-ray device, a tomography (CT) scanning device, a nuclear magnetic resonance (MRI) device, a fundus camera and an ultrasound device.

11. The smart health management system (1) for the telemedicine service according to claim 1, wherein the medical examination and equipment management module (13) is provided by at least one medical examination and equipment supplier from all over the world.

12. The smart health management system (1) for the telemedicine service according to claim 1, wherein the integrated electronic medical record and artificial intelligence analysis data module (112) is installed in one of a wired data storage device and a wireless data storage device, and each of the medical control module (111), the telemedicine diagnosis service module (121), the medical examination and equipment management module (13) and the drug preparation and supply module (122) comprises a memory card with a wireless data transmission function, wherein if the integrated electronic medical record and artificial intelligence analysis data module (112) is installed in the wireless data storage device, the autonomous health management information (S1), the medical diagnosis information (S2), the pathological inspection and imaging examination information (S3) and the drug prescription information (S4) are wirelessly transmitted to the wireless data storage device.

13. The smart health management system (1) for the telemedicine service according to claim 12, wherein the medical control module (111) comprises a main control unit (1111) for controlling each of the integrated electronic medical record and artificial intelligence analysis data module (112), the telemedicine diagnosis service module (121), the medical examination and equipment management module (13), the drug preparation and supply module (122) and the insurance service module (S4), and the medical control module (111) is installed in one of the wired data storage device and the wireless data storage device.

14. The smart health management system (1) for the telemedicine service according to claim 1, wherein the integrated electronic medical record and artificial intelligence analysis data module (112) uses an artificial intelligence technology to analyze, integrate or identify at least one of the autonomous health management information (S1), the medical diagnosis information (S2), the pathological inspection and imaging examination information (S3) and the drug prescription information (S4) so as to generate the personalized medical analysis information (S5) automatically.

15. The smart health management system (1) for the telemedicine service according to claim 1, wherein the electronic medical record (E) is implemented by using a block chain technology.

16. The smart health management system (1) for the telemedicine service according to claim 1, wherein the medical care and sharing information (S6) is provided to the at least one user (P1) and/or at least one additional user (P2) for use, wherein the at least one additional user (P2) is selected and designated by the at least one user (P1).

17. The smart health management system (1) for the telemedicine service according to claim 16, wherein the at least one user (P1) authorizes the at least one additional user (P2) to use the electronic medical record (E) in whole or in part.

18. The smart health management system (1) for the telemedicine service according to claim 16, wherein the at least one user (P1) has a highest management right over the electronic medical record (E), and the at least one user (P1) completely or partially restricts each of the telemedicine diagnosis service module (121), the medical examination and equipment management module (13), the drug preparation and supply module (122) and the insurance service module (S4) to use the electronic medical record (E) in whole or in part.

19. The smart health management system (1) for the telemedicine service according to claim 1, wherein the virtual digital medical institution operation module (120) at least comprises at least one of an appointment consultation and examination management function, a customer relationship management (CRM) function and a marketing function so as to support the at least one online medical institution to perform the telemedicine diagnosis service.

20. The smart health management system (1) for the telemedicine service according to claim 1, wherein at least one of the medical control module (111), the telemedicine diagnosis service module (121), the medical examination and equipment management module (13), the drug preparation and supply module (122) and the insurance service module (S4) has an equipment failure detection function of automatically notifying the at least one user (P1) of an equipment failure information (S8).

21. The smart health management system (1) for the telemedicine service according to claim 1, wherein at least one of the medical control module (111), the telemedicine diagnosis service module (121), the medical examination and equipment management module (13), the drug preparation and supply module (122) and the insurance service module (S4) has an Internet of Things (loT) function.

22. The smart health management system (1) for the telemedicine service according to claim 1, wherein the medical diagnosis information (S2), the pathological inspection and imaging examination information (S3) and the drug prescription information (S4) contain a medical diagnosis cash flow information (S20), a pathological inspection and imaging examination cash flow information (S30) and a drug prescription cash flow information (S40), respectively.

23. A smart health management system (1) for a telemedicine service, **characterized in that** the smart health management system (1) comprises:
a personalized medical management module (11) generating at least one of a medical diagnosis service request (R1), an autonomous health management information (S1), a medical care and information sharing request (R2) and a medical insurance request (R3);
a telemedicine service module (12) electrically connected with the personalized medical management module (11), wherein the telemedicine service module (12) performs a telemedicine diagnosis service and generates a medical diagnosis information (S2) in response to at least one of the medical diagnosis service request (R1) and the autonomous health management information (S1), or the telemedicine service module (12) provides a medical care and sharing information (S6) in response to the medical care and information sharing request (R2);
a medical examination and equipment management module (13) electrically connected with the personalized medical management module (11) and the telemedicine service module (12), wherein the medical examination and equipment management module (13) supports the telemedicine diagnosis service to generate a pathological inspection and imaging examination information (S3) and allows the telemedicine service module (12) to generate the medical diagnosis information (S2), wherein the personalized medical management module (11) stores at least one of the autonomous health management information (S1), the medical diagnosis information (S2), the pathological inspection and imaging examination information (S3) and the drug prescription information (S4) and generates a personalized medical analysis information (S5) automatically, wherein an electronic medical record (E) is automatically generated according to at least one of the autonomous health management information (S1), the medical diagnosis information (S2), the pathological inspection and imaging examination information (S3), the drug prescription information (S4), the personalized medical analysis information (S5) and the medical care and sharing information (S6); and
an insurance service module (S4) electrically connected with the personalized medical management module (11), the medical examination and equipment management module (13) and the telemedicine service module (12), wherein the insurance service module (S4) generates an insurance claim information (S7) automatically in response to the medical insurance request (R3) and the electronic medical record (E) and in response to a cash flow information provided by at least one of the medical examination and equipment management module (13) and the telemedicine service module (12).

24. The smart health management system (1) for the telemedicine service according to claim 23, wherein the personalized medical management module (11) at least comprises:
a medical control module (111) generating at least one of the medical diagnosis service request (R1), the autonomous health management information (S1), the medical care and information sharing request (R2) and the medical insurance request (R3); and
an integrated electronic medical record and artificial intelligence analysis data module (112) electrically connected with the medical control module (111), the telemedicine service module (12) and the medical examination and equipment management module (13), wherein the integrated electronic medical record and artificial intelligence analysis data module (112) stores at least one of the autonomous health management information (S1), the medical diagnosis information (S2), the pathological inspection and imaging examination information (S3) and the drug prescription information (S4) and generates the personalized medical analysis information (S5) automatically, or at least one of the telemedicine service module (12) and the integrated electronic medical record and artificial intelligence analysis data module (112) provides a medical care and sharing information (S6) in response to the medical care and information sharing request (R2) from the medical control module (111), wherein the electronic medical record (E) is automatically generated according to at least one of, the medical diagnosis information (S2), the pathological inspection and imaging examination information (S3), the drug prescription information (S4), the personalized medical analysis information (S5) and the medical care and sharing information (S6).

25. The smart health management system (1) for the telemedicine service according to claim 24, wherein the medical control module (111) generates at least one of the medical diagnosis service request (R1), the medical care and information sharing request (R2) and the medical insurance request (R3) in response to a demand instruction information (I) of at least one user (P1) and/or in response to a personalized basic physiological information description provided by the at least one user (P1) that at least includes a personal symptom, a personal medical history and a personal family history, or the medical control module (111) generates the autonomous health management information (S1) by inputting a physiological characteristic information (S11) of the at least one user (P1) that is measured or monitored through a physiological feature detection device (15).

26. The smart health management system (1) for the telemedicine service according to claim 24, wherein the medical control module (111) and the at least one user (P1) are in wired or wireless communication with each other through a fixed computer or communication device, or the medical control module (111) and the at least one user (P1) are in wired or wireless communication with each other through a portable computer or communication device.

27. The smart health management system (1) for the telemedicine service according to claim 24, wherein the at least one user (P1) is in communication between the medical control module (111) and the telemedicine service module (12) to perform the telemedicine diagnosis service through a network video and communication software.

28. The smart health management system (1) for the telemedicine service according to claim 24, wherein the integrated electronic medical record and artificial intelligence analysis data module (112) is installed in one of a wired data storage device and a wireless data storage device, and each of the medical control module (111), the telemedicine service module (12), the medical examination and equipment management module (13) and the drug preparation and supply module (122) comprises a memory card with a wireless data transmission function, wherein if the integrated electronic medical record and artificial intelligence analysis data module (112) is installed in the wireless data storage device, the autonomous health management information (S1), the medical diagnosis information (S2), the pathological inspection and imaging examination information (S3) and the drug prescription information (S4) are wirelessly transmitted to the wireless data storage device.

29. The smart health management system (1) for the telemedicine service according to claim 24, wherein the integrated electronic medical record and artificial intelligence analysis data module (112) uses an artificial intelligence technology to analyze, integrate or identify at least one of the autonomous health management information (S1), the medical diagnosis information (S2), the pathological inspection and imaging examination information (S3) and the drug prescription information (S4) so as to generate the personalized medical analysis information (S5) automatically.

30. The smart health management system (1) for the telemedicine service according to claim 23, wherein the telemedicine service module (12) at least comprises:
a telemedicine diagnosis service module (121) electrically connected with the personalized medical management module (11), wherein the telemedicine diagnosis service module (121) performs the telemedicine diagnosis service and generates the medical diagnosis information (S2) in response to at least one of the medical diagnosis service request (R1) and the autonomous health management information (S1); and
a drug preparation and supply module (122) electrically connected with the telemedicine diagnosis service module (121), wherein the drug preparation and supply module (122) generates the drug prescription information (S4) in response to the medical diagnosis information (S2), and the drug prescription information (S4) is provided to the telemedicine diagnosis service module (121) and the personalized medical management module (11) for use.

31. The smart health management system (1) for the telemedicine service according to claim 30, wherein the at least one online medical institution in the telemedicine diagnosis service module (121) includes at least one of an online specialist clinic, an online hospital and an online doctor individual organization from all over the world.

32. The smart health management system (1) for the telemedicine service according to claim 23, wherein the medical examination and equipment management module (13) at least comprises at least one of a pathological inspection device (131) and an imaging examination device (132).

33. The smart health management system (1) for the telemedicine service according to claim 23, wherein the medical examination and equipment management module (13) is provided by at least one medical examination and equipment supplier from all over the world.

34. The smart health management system (1) for the telemedicine service according to claim 23, wherein the medical care and sharing information (S6) is provided to the at least one user (P1) and/or at least one additional user (P2) for use, wherein the at least one additional user (P2) is selected and designated by the at least one user (P1).

35. The smart health management system (1) for the telemedicine service according to claim 34, wherein the at least one user (P1) authorizes the at least one additional user (P2) to use the electronic medical record (E) in whole or in part.

36. The smart health management system (1) for the telemedicine service according to claim 34, wherein the at least one user (P1) has a highest management right over the electronic medical record (E), and the at least one user (P1) completely or partially restricts each of the telemedicine service module (12), the medical examination and equipment management module (13) and the insurance service module (S4) to use the electronic medical record (E) in whole or in part.

37. The smart health management system (1) for the telemedicine service according to claim 34, wherein the medical diagnosis information (S2), the pathological inspection and imaging examination information (S3) and the drug prescription information (S4) contain a medical diagnosis cash flow information (S20), a pathological inspection and imaging examination cash flow information (S30) and a drug prescription cash flow information (S40), respectively.

38. The smart health management system (1) for the telemedicine service according to claim 23, wherein the smart health management system (1) for the telemedicine service further comprises a virtual digital medical institution operation module (120), wherein the virtual digital medical institution operation module (120) is electrically connected with the telemedicine service module (12), and the virtual digital medical institution operation module (120) supports at least one online medical institution in the telemedicine service module (12) to perform the telemedicine diagnosis service.

39. A smart health management method for a telemedicine service, **characterized in that** the smart health management method at least comprises steps of:
providing at least one of a medical diagnosis service request (R1), an autonomous health management information (S1), a medical care and information sharing request (R2) and a medical insurance request (R3);
performing a telemedicine diagnosis service and generating a medical diagnosis information (S2) and a drug prescription information (S4) in response to at least one of the medical diagnosis service request (R1) and the autonomous health management information (S1), or providing a medical care and sharing information (S6) in response to the medical care and information sharing request (R2);
supporting the telemedicine diagnosis service to generate a pathological inspection and imaging examination information (S3);
generating a medical diagnosis information (S2) in response to the pathological inspection and imaging examination information (S3);
generating a personalized medical analysis information (S5) in response to at least one of the autonomous health management information (S1), the medical diagnosis information (S2), the pathological inspection and imaging examination information (S3) and the drug prescription information (S4), and automatically generating an electronic medical record (E) according to at least one of the autonomous health management information (S1), the medical diagnosis information (S2), the pathological inspection and imaging examination information (S3), the drug prescription information (S4), the personalized medical analysis information (S5) and the medical care and sharing information (S6); and
automatically generating an insurance claim information (S7) in response to the medical insurance request (R3) and the electronic medical record (E).

40. The smart health management method for the telemedicine service according to claim 39, wherein at least one of the medical diagnosis service request (R1), the medical care and information sharing request (R2) and the medical insurance request (R3) is generated in response to a demand instruction information (I) of at least one user (P1) and/or in response to a personalized basic physiological information description provided by the at least one user (P1) that at least includes a personal symptom, a personal medical history and a personal family history, or the autonomous health management information (S1) is generated by measuring or monitoring a physiological characteristic information (S11) of the at least one user (P1).

41. The smart health management method for the telemedicine service according to claim 40, wherein the at least one user (P1) performs the telemedicine diagnosis service through a network video and communication software.

42. The smart health management method for the telemedicine service according to claim 41, wherein the telemedicine diagnosis service is provided by at least one of an online specialist clinic, an online hospital and an online doctor individual organization from all over the world.

43. The smart health management method for the telemedicine service according to claim 41, wherein the pathological inspection and imaging examination information (S3) is provided by at least one medical examination and equipment supplier from all over the world.

44. The smart health management method for the telemedicine service according to claim 39, wherein the medical diagnosis service request (R1) includes a real-time geographic location that reflects a location of the at least one user (P1).

45. The smart health management method for the telemedicine service according to claim 39, wherein the smart health management method further comprises a step of proactively searching for the at least one user (P1) that is located in a specific area and proactively communicating with the at least one use.

46. The smart health management system (1) for the telemedicine service according to claim 39, wherein the smart health management system (1) further comprises a step of using an artificial intelligence technology to analyze, integrate or identify at least one of the autonomous health management information (S1), the medical diagnosis information (S2), the pathological inspection and imaging examination information (S3) and the drug prescription information (S4) so as to generate the personalized medical analysis information (S5) automatically.

47. The smart health management method for the telemedicine service according to claim 39, wherein the electronic medical record (E) is implemented by using a block chain technology.

48. The smart health management method for the telemedicine service according to claim 39, wherein the medical care and sharing information (S6) is provided to the at least one user (P1) and/or at least one additional user (P2) for use, wherein the at least one additional user (P2) is selected and designated by the at least one user (P1).

49. The smart health management method for telemedicine according to claim 48, wherein the at least one user (P1) authorizes the at least one additional user (P2) to use the electronic medical record (E) in whole or in part.

50. The smart health management method for the telemedicine service according to claim 48, wherein the at least one user (P1) has a highest management right over the electronic medical record (E), and the at least one user (P1) completely or partially restricts a use access right and/or a modification right of the at least one additional user (P2) on the electronic medical record (E).
